# EUROPEAN PATENT APPLICATION

(11) **EP 0 795 537 A1**
(43) Date of publication of application: **17.09.1997**
(21) Application number: 97102655.4
(22) Date of filing: 19.02.1997
(51) Int. Cl.: C07C 67/26, C07C 69/16, C07D 301/12, C07D 303/04

(54) **Process for the synthesis of 2-butene -1,4-diesters**

(30) Priority: 12.03.1996 IT MI960476
(71) Applicant: ENICHEM S.p.A., 20124 Milano (IT)
(72) Inventor: Querci, Cecilia, Novara (IT); Bianchi, Daniele, Arese, (Milano) (IT); Santi, Roberto, Novara (IT); Ricci, Marco, Novara (IT); Soattini, Sergio, Cerano, (Novara) (IT); Ungarelli, Raffaele, Trecate, (Novara) (IT)
(74) Representative: Gennari, Marco

(57) **Abstract**

Process for the synthesis of 2-butene-1,4-diesters starting from 1,3-butadiene which comprises:
a) the epoxidation, at pH of between 5 and 7, of a double bond of butadiene, by reaction with H₂O₂, in the presence of titanium silicalite as catalyst; and
b) the regioselective esterification of the monoepoxide thus obtained.

## Description

The present invention relates to a process for the synthesis of 2-butene-1,4-diesters.

More specifically the present invention relates to a process for the synthesis of 2-butene-1,4-diesters starting from 1,3-butadiene.

2-butene-1,4-diesters are products which are known in literature and can be used as intermediates in numerous organic syntheses. In particular 2-butene-1,4-diesters can be used as intermediates for the synthesis of 1,4-butandiol, a monomer for the production of polyols, polyesters and polyurethane resins, or a further intermediate for the production of other organic compounds such as, for example, tetrahydrofuran.

In fact, 2-butene-1,4-diesters having general formula 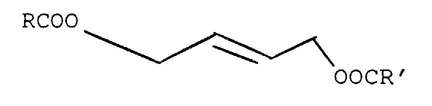 wherein R and R', the same or different, are C₁-C₁₀ alkyl or isoalkyl radicals or C₆-C₁₂ aromatics, can be easily hydrogenated and subsequently hydrolyzed, with conventional techniques, to give 1,4-butandiol:

HO-(CH₂)₄-OH

One of the most well-known processes for the production of 1,4-butandiol is described in Ullmann's Encyclopedia of Industrial Chemistry, Fifth Edition, vol. A4, page 455. According to this process 1,4-butandiol is prepared by the hydrogenation of 2-butino-1,4-diol in turn prepared by reaction between acetylene and formaldehyde.

As this process uses a raw material which is difficult to treat, such as acetylene, various alternative processes have been proposed in literature. An example is described in Italian patent application MI92A 001785 which discloses the preparation of 1,4-butandiol by the regioselective hydrogenation of the corresponding diepoxide (1,2,3,4-diepoxybutane).

An alternative method for the production of 1,4-butandiol could be represented by the hydrogenation and subsequent hydrolysis of 2-butene-1,4-diesters. This method however has not been followed so far as there are no known processes which allow the synthesis of these intermediates with high yields and selectivities.

The purpose of the present invention is to provide a new process for the preparation of 2-butene-1,4-diesters in which yields and selectivities are sufficiently high as to make the process interesting from an industrial point of view.

The present invention therefore relates to a process for the synthesis of 2-butene-1,4-diesters starting from 1,3-butadiene which comprises:
a) the epoxidation, at pH of between 5 and 7, of a double bond of butadiene, by reaction with hydrogen peroxide in the presence of titanium silicalite as catalyst; and
b) the regioselective esterification of the monoepoxide thus obtained.

According to the present invention, the epoxidation of the double bond of butadiene takes place by reaction with hydrogen peroxide in an aqueous solution at 10-60% by weight in a monophase or biphase aqueous-organic system. In the latter case organic solvents, in addition to water, are used as solvents, selected from alcohols such as methanol, ethanol or t-butanol; ketones such as acetone or methyl-t-butylketone; ethers such as methyl-t-butylether (MTBE) or tetrahydrofuran (THF); esters such as methylacetate or ethylacetate (AcOEt); acetonitrile; chlorinated solvents such as dichloromethane, 1,2-dichloroethane (DCE) or 1,1,2,2-tetrachloroethane; etc. The ratio in volume water/organic solvent is generally between 0.01 and 1.

The monoepoxidation catalyst consists of titanium silicalite, a synthetic zeolite containing titanium, silicon and oxygen and corresponding to the formula:

xTiO₂·(1-x)SiO₂

wherein x is between 0.0001 and 0.04. Details on titanium silicalite can be found in Belgian patent 886.812.

The epoxidation reaction of the double bond of butadiene is carried out under such conditions as to minimize the production of by-products such as 1-butene-3,4-diol or diepoxides.

In particular the monoepoxidation reaction is carried out in an acid environment maintaining the pH of the reaction mass at between 5 and 7. This result can be obtained by adding alkaline aqueous solutions to the reaction environment, for example based on NaOH, KOH or NH₄OH, or acid aqueous solutions, for example based on H₂SO₄, H₃PO₄ or CH₃COOH.

The epoxidation reaction, moreover, is carried out at a temperature of between 20 and 60°C, at atmospheric pressure, using from 0.001 to 1 mole of hydrogen peroxide per gram of catalyst. The reaction times are generally between 0.5 and 10 hours.

The molar ratio H₂O₂/butadiene is between 0.05 and 1.5 or, preferably, between 0.1 and 0.5.

The butadiene monoepoxide obtained is recovered from the reaction system with the known techniques, for example by distillation, and esterified with anhydrides having the general formula:

RR'(CO)₂O

wherein R and R', the same or different, represent C₁-C₁₀ (iso)alkyl radicals or C₆-C₁₂ aromatics.

Acetic, propionic, butyric and pivalic anhydrides have proved to be particularly suitable.

The esterification reaction is carried out in an inert atmosphere, in a monophase system in the presence of a catalyst based on palladium. More specifically, the catalyst consists of a complex of palladium which is normally prepared in situ starting from a precursor containing palladium and a suitable ligand. Among the possible precursors, palladium bis(dibenzylideneacetone) is preferred, a compound which is sufficiently stable and easy to handle even though it is not capable, as such, of catalyzing the esterification reaction.

The ligand used is usually a compound containing one or more elements of group V of the periodic table, preferably phosphorous or nitrogen. In particular, arylphosphines can be used as ligands, preferably diphenylmethylphosphine (PPh₂Me) and 1,10-phenanthroline, also substituted.

The ligand is used in a molar ratio with the palladium (metal) of between 1 and 10, preferably between 2 and 6.

The reaction solvent can be selected from those used for the monoepoxidation reaction or from hydrocarbon solvents such as hexane, heptane, cyclohexane, etc.; from oxygenated solvents, such as tetrahydrofuran, dioxane, ethyl acetate, etc.; or from chlorinated solvents such as methylene chloride, dichloroethane, etc.

The concentration of monoepoxide in the solvent varies from 1 to 20% by weight, preferably from 1 to 10% whereas the catalyst is used in such a way that the molar ratio monoepoxide/palladium is between 10 and 10,000, preferably between 10 and 5,000.

The esterification reaction takes place at atmospheric pressure and at a temperature between 10 and 100°C, preferably between 15 and 40°C. The reaction times are generally between 5 minutes and 2 hours.

The molar ratio between the anhydrides and the monoepoxide is between 0.5 and 4, preferably between 0.75 and 1.25.

Some illustrative but non-limiting examples are provided for a better understanding of the present invention and for its embodiment.

### EXAMPLE 1

The operation was carried out with a jacketed glass cylindrical reactor with a volume of 150 ml, equipped with a thermostat-regulated heating/cooling bath, temperature control, electrode and pH measurer of the Amel type, mechanical stirrer, reflux condenser cooled by a bath thermostat-regulated at -25°C, feeding inlet of the solution of H₂O₂ and immersed glass tube for the feeding of the gaseous butadiene.

0.75 g of titanium silicalite having a titer, expressed as titanium, of 93%, 12 ml of demineralized water and 100 ml of ethanol were charged. The mixture was heated to 40°C correcting the pH to 6.5 by adding an aqueous solution at 5% of KOH dropwise.

The feeding of the substrate and H₂O₂ was started at 40°C, and under vigorous stirring (850 rpm), maintaining the pH at 6.5 with periodical additions of KOH solution. 1.5 g of H₂O₂ (aqueous solution at 60% by weight diluted with 1.5 ml of ethanol), equal to 27 mmoles were fed over a period of 10 minutes, whereas during the following 20 minutes the feeding of 1,3-butadiene, equal to 7.6 g (133 mmoles) was completed. The increases of pH were corrected to 6.5 with the addition of an 0.1 N aqueous solution of sulfuric acid.

The reaction continued for a further hour at 40°C and pH 6.5.

At the end, the mixture was filtered under nitrogen pressure to remove the catalyst (washed with ethanol) the filtrate being collected in a container cooled with a water and ice bath. 134.01 g of solution were obtained with a titer of 0.63% of 1,2-epoxy-3-butene, equal to 0.85 g (12.1 mmoles), with a yield of 9.1% of diene and 44.8% of hydrogen peroxide. The selectivity was equal to 68.7% (referring to the hydrogen peroxide).

Gaschromatographic analysis was carried out on a Carlo Erba Mega gaschromatograph Series 5300, using a capillary column of the type PTE-5, 30 metres long and using n-decane as internal standard. Analysis of the residual hydrogen peroxide at the end of the reaction was carried out by iodometric titration. 0.32 g of H₂O₂ equal to 9.4 mmoles (35% of oxidant charged) were found.

### EXAMPLE 2

Example 1 was repeated with an overall reaction time of 4 hours. A yield of 64% and a selectivity of 64.7% (referring to the hydrogen peroxide) were obtained.

### EXAMPLES 3-7

Operating as described in example 1, the following examples were carried out substituting ethanol with other solvents miscible with water shown in Table 1.

**TABLE 1**

| Ex. | Solvent | Yield (H₂O₂) % | Select.(H₂O₂) % |
|---|---|---|---|
| 3 | Methanol | 68.6 | 69.3 |
| 4 | t-Butanol | 32.4 | 56.0 |
| 5 | Acetonitrile | 22.2 | 52.2 |
| 6 | Tetrahydrofuran | 12.7 | 29.2 |
| 7 | Acetone | 21.7 | 33.4 |

### EXAMPLES 8-11

Operating as described in example 1, the following examples were carried out substituting ethanol with other solvents partially miscible with water shown in Table 2.

**TABLE 2**

| Ex. | Solvent | Yield (H₂O₂) % | Select.(H₂O₂) % |
|---|---|---|---|
| 8 | Ethylacetate | 43.8 | 62.0 |
| 9 | Methylacetate | 31.2 | 65.4 |
| 10 | MIBE | 26.7 | 51.2 |
| 11 | Pinacolone | 24.0 | 60.3 |

### EXAMPLE 12

The same procedure was used as in example 1, operating with 1,2-dichloroethane, a solvent immiscible with water. A yield of 47% and a selectivity or 58.8% (again referring to the hydrogen peroxide) were obtained.

### EXAMPLE 13 (Comparative)

The same procedure was used as in example 3 without making any correction of the pH. A yield of 41.3% and a selectivity of 42.2% (referring to hydrogen peroxide) were obtained.

### EXAMPLE 14 (Comparative)

The same procedure was used as in Example 13 using as catalyst the titanium silicalite of example 1 previously treated with an aqueous solution of sodium acetate at 0.5% at reflux temperature, subsequently washed with demineralized water and with methanol, then dried and calcined at 550°C.

A yield of 39.8% and a selectivity of 65%, referring to the hydrogen peroxide, were obtained.

### EXAMPLE 15 (Comparative)

Using the same procedure described in example 9 but regulating the pH to 7.7, a yield of 9.3% and a selectivity of 25.8% were obtained.

### EXAMPLES 16-18

The same procedure was used as in example 15, but with different pH values, as illustrated in Table 3 below.

**TABLE 3**

| Ex. | pH | Yield (H₂O₂) % | Select. (H₂O₂) % |
|---|---|---|---|
| 16 | 7.0 | 19.3 | 59.1 |
| 17 | 6.2 | 39.3 | 59.2 |
| 18 | 5.9 | 47.0 | 58.3 |

### EXAMPLE 19

Example 3 was repeated, charging 29 ml of demineralized water (instead of 12 ml). 19.6 mmoles of 1,2-epoxy-3-butene were obtained, corresponding to a yield of 72.7% and a selectivity of 73.3 % (referring to the hydrogen peroxide.

### EXAMPLE 20

4 mg of palladium bis(dibenzylideneacetone) (0.007 mmoles), 8 microlitres of diphenylmethylphosphine (0.043 mmoles), 1 ml of tetrahydrofuran (THF) and 186 mg of pivalic anhydride (1 mmole) were charged into a flask equipped with magnetic stirring and a tap for the inlet of gas, in an atmosphere of argon. 70 mg (1 mmole) of 1,2-epoxy-3-butene in 2 ml of THF were then fed.

The mixture was maintained under stirring at 25°C for 15 minutes and then analyzed by gaschromatography using 30 mg of dodecane as internal standard.

212.5 mg of 2-butendiol 1,4-dipivalate and 28.2 mg of 3-butendiol 1,2-dipivalate were obtained with yields of 83% and 11% respectively referring to the butadiene monoepoxide charged (ratio 1,4/1,2 = 7.5).

### EXAMPLE 21

Example 20 was repeated substituting the pivalic anhydride with 1 mmole of butyric anhydride. 166.4 mg of 2-butendiol 1,4-dibutyrate and 54.7 mg 3-butendiol 1,2-dibutyrate were obtained with yields of 73% and 24% respectively referring to the butadiene monoepoxide charged (ratio 1,4/1,2 = 3).

### EXAMPLE 22

Example 20 was repeated substituting the diphenylmethylphosphine with an equal number of moles of triphenylphosphine. After 24 hours a conversion of 77% of butadiene monoepoxide and a yield of 33% of 2-butendiol 1,4-dipivalate were obtained, with a ratio 1,4/1,2 = 1.4.

### EXAMPLES 23 - 27

The same procedure was used as in example 20, substituting tetrahydrofuran with the solvents reported in Table 4.

**TABLE 4**

| Ex. | Solvent | Yield butadiene monoepoxide 1,4-diester (%) | Ratio 1,4/1,2 |
|---|---|---|---|
| 23 | AcOEt | 68 | 3.5 |
| 24 | CH₂Cl₂ | 63 | 5.1 |
| 25 | DCE | 64 | 3.9 |
| 26 | CH₃CN | 40 | 1.2 |
| 27 | Me(OCH₂CH₂)₄OMe | 68 | 3.4 |

## Claims

1. A process for the synthesis of 2-butene-1,4-diesters starting from 1,3-butadiene which comprises:
a) the epoxidation, at pH of between 5 and 7, of a double bond of butadiene, by reaction with hydrogen peroxide in the presence of titanium silicalite as catalyst; and
b) the regioselective esterification of the monoepoxide thus obtained.

2. The process according to claim 1, wherein the epoxidation of the double bond of butadiene takes place by reaction with hydrogen peroxide in an aqueous solution at 10-60%.

3. The process according to claim 1 or 2, wherein the epoxidation reaction takes place in the presence of an organic solvent.

4. The process according to claim 3, wherein the volume ratio water/organic solvent is between 0.01 and 1.

5. The process according to any of the previous claims, wherein the monoepoxidation catalyst consists of titanium silicalite corresponding to the formula:
xTiO₂·(1-x)SiO₂
wherein x is between 0.0001 and 0.04.

6. The process according to any of the previous claims, wherein the molar ratio H₂O₂/butadiene is between 0.05 and 1.5.

7. The process according to any of the previous claims, wherein the regioselective esterification of the butadiene monoepoxide is carried out with anhydrides having the general formula:
RR'(CO)₂O
wherein R and R', the same or different, represent C₁-C₁₀ (iso)alkyl radicals or C₆-C₁₂ aromatics.

8. The process according to any of the previous claims, wherein the esterification reaction is carried out in an inert atmosphere, in a monophase system in the presence of a catalyst based on palladium.

9. The process according to any of the previous claims, wherein the molar ratio monoepoxide/palladium is between 10 and 10,000.

10. The process according to any of the previous claims, wherein the molar ratio between the anhydrides and the monoepoxide is between 0.5 and 4.
